(19) Europäisches Patentamt
European Patent Office
Office européen des brevets

(11) Publication number: 0 114 104
B1

(12) # EUROPEAN PATENT SPECIFICATION

(45) Date of publication of patent specification: 19.04.89

(51) Int. Cl.[4]: A 61 K 31/70 // (A61K31/70, 31:195, 31:19)

(21) Application number: 84300179.3

(22) Date of filing: 12.01.84

(54) **An oral energy-rich composition and solution for combatting diarrhea in mammals.**

(30) Priority: 13.01.83 US 457829

(43) Date of publication of application:
25.07.84 Bulletin 84/30

(45) Publication of the grant of the patent:
19.04.89 Bulletin 89/16

(84) Designated Contracting States:
AT BE CH DE FR GB IT LI LU NL SE

(56) References cited:
FR-A-2 252 848
FR-A-2 345 155
FR-A-2 412 313
FR-A-2 467 599

(73) Proprietor: COLORADO STATE UNIVERSITY RESEARCH FOUNDATION, INC.
Fort Collins
Colorado 80523 (US)

(72) Inventor: Phillips, Robert W.
2 Windjammer Cove
Fort Collins Colorado 80524 (US)

(74) Representative: Hale, Stephen Geoffrey et al
J.Y. & G.W. Johnson Furnival House 14/18 High Holborn
London WC1V 6DE (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 114 104 B1

## Description

Field of the invention

Diarrheal diseases in mammals cause severe dehydration and electrolyte-energy imbalances. The present invention provides a composition which is able to restore the electrolytes which have been lost and, in addition, provides a sufficiency of energy substrates in a readily absorbable and utilizable form.

Background of the invention

Several prior investigators have been concerned with diarrheal diseases. These investigators have proposed oral therapeutic intervention to reduce the serious impact of diarrheal diseases. Generally, these systems are directed primarily towards solving the problem of dehydration and, in some cases, towards providing electrolytes and a minimal amount of energy sources such as glucose. However, the principal reason for including organic molecules which might provide some energy has been to enhance absorption. It is generally believed today that oral fluids must be supplied as essentially isoomotic fluids (approximately 300 mOsm/l) since that is the concentration of solute in body fluids. Hyperosmotic fluids are considered detrimental since they are believed to cause increased secretion. This is brought out in a paper by R. J. Bywater entitled: Pathosphysiology and Treatment of Calf Diarrhea, *Proceedings XII World Congress of Diseases of Cattle, Amsterdam, Sept. 1982, pp. 291—297.* In discussing oral therapy for diarrhea, Dr. Bywater reiterated that "solutions for this purpose should be approximately isotonic (300 mOsm/kg) since, before they can be absorbed, hypertonic solutions must first become isotonic". In another paper presented at the same World Congress by C. Demigne, C. Remesy and F. Chartier entitled: Interest of Acetate in Oral Glucose-electrolytes Formulations for Treatment of Dehydration of Diarrheic Calves, *Proceedings XII World Congress of Diseases in Cattle, Amsterdam Sept. 1982, pp. 305—309,* it is stated "Osmolarity is also an important factor, the optimum being possible in the range of 300 to 350 mOsm". However, if an isotonic fluid is given, some benefit is derived, but an insufficient amount of energy or electrolytes or both will be provided to the diarrheic animal. This is a particularly serious problem in young neonates who have a greater tendency to become diarrheic. In another paper in this field. G. Alexander, N. W. Bennett and R. T. Gemmell discuss Brown Adipose Tissue in the Newborn Calf (*Bos taurus J. Physiol. 244:223—234, 1975,* and state that young neonates do not have sufficient energy reserves in their body. In addition, these animals have difficulty in mobilizing the energy that is present.

US—A—3,898,328 describes a dry stable composition for the treatment of scours and dehydration, and US—A—4,164,568 describes an oral scour formulation comprising 40—80% of actively absorbed monosaccharide, 7.5—30% of actively absorbed naturally occurring amino aeid and 0.5 to 10% of citric acid or a citrate. These oral products are intended to provide only 20—30% of the animal's basic maintenance energy requirement and, therefore, will contribute to continuing body weight loss and energy deficit.

In our earlier US—A—3,928,574, a method and composition for treating diarrhea in bovine animals are disclosed. The composition consists of sodium chloride, a potassium salt and glucose in aqueous solution. The solutions are buffered to maintain a basic condition, such that pH does not exceed 10. These solutions are formulated for use intravenously or subcutaneously. The formulation described in this patent does not include acetate for rapid energy and absorption, nor glycine and citric acid.

A prominent clinical sign of diarrhea is acidosis due to intestinal bicarbonate loss, anaerobic metabolism and decreased renal function. Blood bicarbonate levels are often decreased by 50%. The prior art investigators contend that acidosis may be corrected by providing organic acids alone which will be converted to bicarbonate. However, this is not the case, for metabolism of organic acid leads only to the production of carbon dioxide. It is critically necessary to supply sufficient sodium ion or other alkali metal cations as a salt of the organic acid. The metabolism of that acid will provide one bicarbonate for every monovalent cation such as sodium.

For example:

1 citric acid on oxidation→6 $CO_2$

1 acetic acid on oxidation→2 $CO_2$

1 sodium acetate on oxidation →1 $CO_2$+1 sodium bicarbonate ($NaHCO_3$)

Summary of the invention

It has now been found that, contrary to the teaching of the prior investigators, when dealing with oral therapy, a formulation comprising an actively absorbed monosaccharide, an actively absorbed naturally occurring amino acid, citric acid, and a non-toxic alkali metal salt of acetic acid, optionally together with one or more inorganic salts such as sodium chloride and potassium chloride, when prepared in dry form and added to water prior to use, can be used to make a hypertonic oral solution, preferably of from 600 to 1200 mOsm/l or 2 to 4 times normal body osmolality. The formulation provides an adequate easily absorbed metabolized substrate and is intended to be administered at a rate such that the energy substrates it contains provide 50 to 110% of a calf's (or other mammal's) maintenance energy requirements. The major component of this formulation is the monosaccharide as it provides the most usable energy per Osmole.

There is also a significant quantity of a non-toxic alkali metal salt of acetic acid which is most rapidly utilized for immediate energy following absorption and, thus, provides a very immediate energy supply and also an immediate source of bicarbonate. The metabolism of the acetate in the body is over 5 times as fast as the metabolism of glucose on a molar basis and, thus, provides immediate energy as well as bicarbonate. The bicarbonate formed and retained results from the provision of an equivalent amount of a cation with an organic metabolizable anion such as acetate.

Description of the drawings

Figure 1 of the accompanying drawings shows the therapy response based on rectal temperature and fetlock surface temperature as a function of time and plasma glucose concentration;

Figure 2 is a graph of the glucose blood level relating to time after dose and compares the formulation of the current invention with the formulation of the prior art as set forth in US—A—4,164,568; and

Figure 3 is a graph of the changes in blood urea nitrogen during the course of therapy and compares formulation of the current invention with the formulation set forth in US—A—4,164,568.

Figure 4 is a graph showing changes in glucose concentration during 3 hours following therapy.

Detailed description of the invention

The easily absorbed and metabolized composition according to the present invention which is useful in treatment of diarrhea in animals comprises 70 to 90 (more preferably 81 to 85) weight percent of an actively absorbed monosaccharide, 1 to 7.5 (more preferably 2 to 6) weight per cent of an actively absorbed naturally occurring amino acid, 0.1 to 5 (more preferably 0.2 to 0.45) weight percent of citric acid, and 4 to 12 weight percent of a non-toxic alkali metal salt of acetic acid. The concentrations of the first-mentioned and last-mentioned ingredients are preferably such that when the composition is diluted with water for administration, they are present in the resulting aqueous mixture at concentrations of 30 to 90 grams per liter and 3 to 12 grams per liter, respectively. Preferably, the composition also contains 3 to 8 weight percent of inorganic salts such as sodium chloride and potassium chloride.

The invention also provides an aqueous solution for use in the treatment of diarrhea in bovine animals and comprising an actively absorbed monosaccharide such as glucose, an actively absorbed naturally occurring amino acid such as glycine, citric acid and a non-toxic alkali metal salt of acetic acid, for example a solution obtained by diluting a composition according to the invention with water to the concentrations specified above, the solution being one which will have an osmolality of 50 to 100 mOs as oxidised.

Examples of suitable actively absorbed monosaccharides include dextrose and galactose. The actively absorbed naturally occurring, amine acid may for example be glycine or a related compound. Examples of non-toxic alkali metal salts of acetic acid are sodium acetate, potassium acetate and lithium acetate. When these formulations are prepared and administered in the recommended quantities, they provide approximately 80% of the animal's energy requirements. Metabolism of the acetate component will yield a substantial amount of bicarbonate which can rapidly and significantly improve the bicarbonate condition of the diarrheic animal. This is an important factor, since it is widely recognized that diarrheic animals are acidotic with low bicarbonate levels. The glycine, acetate and citric acid together with any electrolytes enhance the absorption of all ingredients because they are absorbed by different mechanisms.

The present invention is illustrated by the following specific but non-limiting examples.

Example I

A formulation was prepared containing the following components:

TABLE I

| | wt.% | Grams | Kcal/two liters | mOsm/l |
|---|---|---|---|---|
| Glucose | 81.8 | 130 | 520 | 361 |
| Glycine | 4.72 | 7.5 | 23 | 50 |
| Na Acetate | 6.20 | 9.84 | 18 | 120 |
| Citric acid | 0.44 | 0.7 | 3 | 2 |
| NaCl | 4.42 | 7.02 | — | 120 |
| KCl | 2.43 | 3.72 | — | 50 |
| Water 2 liters | | | 564 | 703 |

The composition of the residual replacement fluid after metabolism of the organic constituents is set out in the Table below.

TABLE II

| Electrolytes | Fluid from invention | mEq/liter Normal plasma | Diarrheic plasma |
|---|---|---|---|
| Sodium | 120 | 135 | 135 |
| Potassium | 25 | 5 | 8 |
| Chloride | 85 | 100 | 100 |
| Bicarbonate (from acetate) | 60 | 25 | 15 |
| Total | 290 | | |

The therapy therefore provides an extracellular fluid with added potassium and bicarbonate. This is important as most fluid is lost from the extracellular fluid pool.

The hematocrit declined rapidly in the first hour after therapy, and remained lower throughout the test, as is shown in Table III.

TABLE III
Changes after therapy

| | Pre | Hour 1 | Hour 2 | Hour 3—4 |
|---|---|---|---|---|
| Plasma sodium mEq/l | 138.9±0.3 | 140.3±1.0 | 140.5±0.6 | 139.9±1.0 |
| Plasma potassium mEq/l | 5.21±0.20 | 4.59±0.4 | 4.65±1.0 | 4.56±1.2 |
| Hematocrit % | 58.7±1.3 | 51.2±2.4 | 48.9±0.4 | 50.7±1.2 |

These data, when considered with the lack of change in plasma sodium concentration, provide firm evidence that secretion into the gastrointestinal tract and hemoconcentration were not occurring. The animal was hypoglycemic with a very low blood glucose (55 mg/dl) prior to therapy indicating a precarious energy state. Plasma glucose began to increase within 15 minutes of therapy and remained elevated for the duration of the test. The plasma glucose was still at 100 mg/dl four hours after therapy indicating a sustained benefit. Minor hyperkalemia was corrected. This data is confirmed in Table III above. Figure 1 of the drawings is a graphic illustration of the benefit of this therapy. It shows the restoration of normal fluid and energy metabolism as seen by the increase of skin temperature on the extremities without modification of rectal temperature. The incrase in extracellular fluid volume, including blood, allowed for normal blood circulation pattern to be reestablished, and as blood flow to the limbs was increased their temperature increased.

Example II

This example compares the beneficial effects of the formulation set out in Table I with the formulation set out US—A—4,164,568 consisting of the following composition:

TABLE IV
Formulation from US—A—4,164,568

| | wt.% | Grams | Kcal/2 liters | mOsm/l |
|---|---|---|---|---|
| Glucose | 69.9% | 44.6 | 178 | 124 |
| Glycine | 9.7% | 6.2 | 19 | 41 |
| Citric A | 0.8% | 0.5 | 3 | 1 |
| $K_3$ Citrate | 0.2% | 0.1 | — | <1 |
| NaCl | 13.3% | 8.5 | — | 145 |
| $KH_2PO_4$ | 6.4% | 4.1 | — | 30 |
| 2 liters $H_2O$ | | | 200 | 342 |

The composition of the residual replacement fluid after metabolism of the organic constituents is set out in the table below.

TABLE V

| | Fluid from US—A—4,164,568 | m/Eq/liter Normal plasma | Diarrheic plasma |
|---|---|---|---|
| Sodium | 72 | 135 | 135 |
| Potassium | 15 | 5 | 8 |
| Chloride | 72 | 100 | 100 |
| Bicarbonate (from $K_3$ citrate) | <1 | 25 | 15 |
| Phosphate | 15 | 5 mg/100 mls | 5 mg/100 mls |
| Total | 175 | | |

This formulation is a commercially available product and was used according to label directions. Both the formulation set out in Table I and the formulation covered in US—A—4,164,568 were administered as two quart (1.9 liter) doses to neonatel diarrheic calves less than 75 lbs (34 kg) weight and 3 quart (2.85 liter) doses to neonatal calves greater than 75 lbs (34 kg) weight twice a day for two days. After the end of the two-day period, the dosage was changed to a mixture of equal quantities of the therapy fluid with whole cows milk. During the first two days of therapy, when the products were administered alone, the efficacy can be more clearly delineated. One of the princple advantages of the therapy of the instant application is that the hyperosmotic solutions enhance net absorption. This is contrary to current thought as is pointed out above. The effect is shown by following the concentration of stable components in the blood plasma, which reflect this effect. These data are set out in Table VI.

TABLE VI
Changes in plasma protein concentration one hour following therapy

| | Pretherapy | 1 hour post | Decrease |
|---|---|---|---|
| Formulation of Table I | 4.9±0.3 | 4.6±0.4 | −0.3 |
| Formulation of Table IV | 4.8±0.3 | 4.6±0.2 | −0.2 |

If the formulation of the present invention causes proteins to become more concentrated, then net secretion, not net absorption, has occurred. However, the formulation of the present invention, on the average, caused more dilution of plasma proteins in the first hour after administration than the formulation covered in US—A—4,164,568. This phenomenon demonstrates rapid net absorption as further evidence of rapid absorption by the diarrheic calves.

The average increase in plasma glucose at the end of the first hour by the calves given the formulation of the present invention was 104 mg/dl. In contrast, the calves receiving the formulation covered by US—A—4,164,568 had an average increase of only 53 mg/dl. By the end of 3 hours, the average elevation of plasma glucose above pretherapy levels in the calves receiving the formulation of the present invention was 70 mg/dl versus only 20 mg/dl for the therapy using the formulation described in US—A—4,164,568. This is shown graphically in Figure 2 of the drawings. This continuing three-fold elevation emphasizes the sustaining longer term benefits of the formulation of the present invention in supplying much needed readily available energy to the initially energy deficient hypoglycemic calves.

Example III

This example illustrates the further benefit of the increased availability of metabolizable glucose in the therapy of the present invention as compared to the therapy of the formulation covered in the above-identified US patent. This benefit is shown in Table VII.

TABLE VII
Plasma potassium changes due to therapy

| | K (mEq/l) pretherapy | 3 hours following therapy change in K (mEq/l) |
|---|---|---|
| Formulation of Table I | 5.3±0.6 | −1.1±0.5 |
| Formulation of Table IV | 5.0±0.4 | −0.1±0.3 |

All animals were hyperkalemic prior to therapy. Three hours following the therapy the decrease in plasma potassium averaged 1.1±0.5 mEq/l for the calves receiving the therapy of the formulation of the present invention and all were within the normal range. In contrast, the calves receiving the therapy covered in the US Patent set out above had only minimal 0.1±0.3 mEq/l decrease, and several remained hyperkalemic.

In addition, the mean blood urea nitrogen decreased precipitously following the initiation of the treatment of the present application. Prior to the first therapy, the blood urea nitrogen was 35 mg/dl and was in the normal range at 9 mg/dl for 1½ days prior to the 4th therapy. This information is shown in Figure 3 of the drawings. A decrease of blood urea nitrogen of this magnitude may be due to enhanced renal function allowing a more normal excretory pattern or may be due to a decrease in the catabolism of protein due to the provision of ample energy substrates. All calves treated with the formulation of the present invention had blood urea nitrogen levels in a normal range in the last one-half of the experiment. Figure 3 graphically compares the changes in the blood urea nitrogen during the course of therapy with the formulation of the current invention and with the formulation of US—A—4,164,568.

One of the best indications of the long-term benefit of the therapeutic approach of the present invention is the maintenance of body weight during therapy. These data are set out in Table VIII.

TABLE VIII
Change in body weight (kg) following therapy

| | Formulation of Table I | Formulation of Table IV |
|---|---|---|
| 2 days* | +0.68 | 0 |
| 4 days** | +2.04 | −0.45 |

* received therapy alone
** sum of all 4 days: in the last two days received 50% whole milk and 50% therapy.

When the calves received the therapy of the present invention alone, the calves gained some weight. The gain was more pronounced in the last 2 days when the formulation was changed to 50% milk and 50% of the formulation in Table I. In this case, the animals received energy for maintenance as well as growth. In the first two days, it is likely that the gain in weight was due to rehydration without body wasting. The formulation of the prior art undoubtedly had some rehydration which was masked due to the energy deficit of the therapy and no weight change was seen.

Example IV

In mildly diarrheic calves, over the entire testing period, potassium concentration changes were evaluated one hour following therapy. Based on six evaluations on each calf they were slightly hyperkalemic prior to therapy shown in Table VIII. One hour following the invention therapy potassium level of all calves had dropped to within the normal range. The average decrease was 0.6 mEq/l.

The data obtained are set out in Table IX below.

TABLE IX
Potassium concentration (mEq/l) changes due to therapy

| | Pretherapy | 1 hour post | Mean change |
|---|---|---|---|
| Invention | 5.1±0.1 | 4.5±0.1 | −0.6 |
| Prior art US—A—4,164,568 | 5.0±0.1 | 4.8±0.1 | −0.2 |

In the calves treated with the prior art formulation, the decrease in K was considerably less. On several

occasions these mammals remained hyperkalemic. These changes were seen in spite of the higher potassium concentration in these treated with the formulation of the present invention.

Example V

Prior to any therapy administration, all of the mildly diarrheic calves had low-normal blood glucose concentration averaging 78 and 71 mg/dl. In Figure 4 of the drawings, changes in glucose concentration during the three hours following therapy have been plotted for calves treated with the formulation of the present invention and those treated in the formulation of the prior art for a two-period period, or four treatments per calf, during the period of therapy alone, when the calves were receiving less than maintenance energy requirements. The calves treated with the formulation of the present invention became slightly hypoglycemic between therapies, dropping from a mean of 78 to a mean of 67 mg/dl. The calves treated with the prior formulation became severely hypoglycemic; they had entered the study with a mean blood glucose of 71 mg/dl, their pretherapy glucose concentration was 49 mg/dl during the time that they received the therapy.

The formulation of the present invention produced a significantly greater plasma glucose response. Based on the area under the curve it was twice as great. Further, the calves treated with the formulation of the present invention had high normal glucose concentration three hours following therapy while the prior art calves had already become hypoglycemic again (Figure 1).

Example VI

Further evidence substantiating the claim that the hyperosmotic nature of the formulation of the present invention does not cause hemoconcentration can be gleaned from Table X below which represents the average change in hematocrit and plasma protein concentration one hour following therapy in mildly diarrheic calves treated with the formulation of the present invention. On the average, the therapy elicited a minor hemodilution as evidenced by a decrease in both hematocrit and plasma protein concentrations. This establishes that the invention, in spite of its hyperosmolar natures does not cause excessive fluid secretion from the blood.

Example VII

The effect of the therapy on blood gas and acid base status in mildly diarrheic calves is presented in Table XI below compared to prior art therapy. Although numerous reports attest to the prevalence of acidosis and decreased bicarbonate levels in more severe diarrhea, these mildly diarrheic calves had normal blood pH an essentially normal blood bicarbonate and base excess values. The calves treated with the formulation of the present invention had a mean increase of 0.023 pH units compared to no change or a slight decrease (−0.005) pH units in the animals treated with the formulation of the prior art. They, in addition, with the formulation of the prior art had a decrease in total bicarbonate and no change in base excess. Thus, the therapy of the present invention was able to change all of these important parameters of acid base status in a beneficial direction. That is, it increased alkalinity which is of important significance in treating diarrheic acidosis.

TABLE X
Therapy induced changes in hematocrit and plasma protein concentrations

| | Therapy | # Calves | Pre-therapy | 1 Hr. post | Pre-therapy | 1 Hr. post |
|---|---|---|---|---|---|---|
| Day one | 1 | 3 | 24.5 | 24.5 | 4.5 | 4.6 |
| | 2 | 3 | 28.7 | 29.3 | 4.4 | 4.5 |
| Day two | 3 | 3 | 30.0 | 28.7 | 4.4 | 4.5 |
| | 4 | 3 | 29.3 | 28.7 | 4.5 | 4.4 |
| Day three | 5 | 3 | 30.3 | 28.7 | 5.2 | 4.7 |
| | 6 | 3 | 26.7 | 27.0 | 4.8 | 4.6 |
| Average | | | 29.2 | 27.0 | 4.8 | 4.5 |

TABLE XI
Acid base changes due to therapy*

| | pH invention | US—A—4,164,568 prior art therapy |
|---|---|---|
| Pre therapy | 7.461±0.007 | 7.470±0.008 |
| Post therapy (1 hour) | 7.484±0.012 | 7.465±0.005 |
| Change in pH | +0.023 | −0.005 |

Total bicarbonate

| | Invention | Prior art therapy |
|---|---|---|
| Pre therapy | 24.9±0.5 | 21.8±0.5 |
| Post therapy (1 hour) | 26.0±0.5 | 21.1±0.4 |
| Change in $HCO_3$ | +1.1 | −0.7 |

Base excess**

| | Invention | Prior art therapy |
|---|---|---|
| Changes in base excess 1 hour after therapy | +1.5±0.3 | +0.1±0.1 |

* Values shown are mean ±S.E.
** Base excess is a measure of total acidity and alkalinity. An increase in base excess indicates correction of acidosis.

**Claims**

1. An easily absorbed and metabolized veterinary composition useful for the treatment of diarrhea in mammals and containing a major proportion of an actively absorbed monosaccharide and minor proportions of an actively absorbed naturally occuring amino acid and of citric acid, characterized in that the composition additionally contains 4 to 12 weight percent of non-toxic alkali metal salt of acetic acid, the monosaccharide concentration being 70 to 90 weight percent, the amino acid concentration being 1 to 7.5 weight percent and the citric acid concentration being 0.1 to 5 weight percent, all based on dry weight.

2. A veterinary composition according to Claim 1, characterized in that it is dissolved in an aqueous carrier suitable for veterinary administration such that the monosaccharide is present in a concentration of 30 to 90 grams per liter.

3. A veterinary composition according to Claim 2, characterized in that the osmolality of the fluid is from 600 to 1,200 mOsm per liter.

4. A veterinary composition according to Claim 2, or 3, characterized in that the non-toxic alkali metal salt of acetic acid is provided in an amount such that the bicarbonate formed from the acetate as it is oxidized will be from 40 to 120 mOsm per liter.

5. A veterinary composition according to any of Claims 1 to 4, characterized in that the alkali metal salt of acetic acid is sodium acetate.

6. A veterinary composition according to any of Claims 1 to 5, characterized in that sodium and potassium ions are present, the sum of the sodium and potassium present is 120 to 160 mOsm/l with the potassium being present at 10 to 30 mOsm/l and the sodium at 110 to 150 mOsm/l.

7. A veterinary composition according to any of Claims 1 to 6, characterized in that the energy substrates consisting of the monosaccharide, the amino acid, the citric acid and the acetate present provide 50 to 100 percent of the animal's energy requirements as they are metabolized.

8. A veterinary composition according to Claim 7, characterized in that as the energy substrates are metabolized the remaining ions approximate an isoosmotic extracellular fluid.

9. A veterinary composition useful for treating diarrhea in bovine animals and comprising an aqueous solution of an actively absorbed monosaccharide, an actively absorbed naturally occurring amino acid and citric acid, characterized in that it additionally contains a non-toxic alkali metal salt of acetic acid and will have an osmolality of 50 to 100 mOs as oxidized.

10. A veterinary composition according to any of Claims 1 to 9, characterized in that the monosaccharide is glucose and the amino acid is glycine.

**Patentansprüche**

1. Leicht resorbierte und metabolisierte Veterinärzusammensetzung zur Verwendung bei der Behandlung von Diarrhoe bei Säugetieren, enthaltend einen grösseren Anteil eines aktiv resorbierten Monosaccharids und kleinere Anteile einer aktiv resorbierten, natürlich vorkommenden Aminosäure sowie Zitronensäure, dadurch gekennzeichnet, dass die Zusammensetzung ferner 4 bis 12 Gewichtsprozent eines nicht-toxischen Alkalisalzes der Essigsäure enthält, wobei die Monosaccharidkonzentration 70 bis 90 Gewichtsprozent, die Aminosäurekonzentration 1 bis 7,5 Gewichtsprozent und die Zitronensäurekonzentration 0,1 bis 5 Gewichtsprozent beträgt, jeweils auf das Trockengewicht bezogen.

2. Veterinärzusammensetzung nach Anspruch 1, dadurch gekennzeichnet, dass sie in einem wässrigen, zur tierärztlichen Verabreichung geeigneten Träger so gelöst ist, dass das Monosaccharid in einer Konzentration von 30 bis 90 Gramm pro Liter vorliegt.

3. Veterinärzusammensetzung nach Anspruch 2, dadurch gekennzeichnet, dass die Osmolatität der Flüssigkeit 600 bis 1200 mOsm pro Liter beträgt.

4. Veterinärzusammensetzung nach Anspruch 2 oder 3, dadurch gekennzeichnet, dass das nichttoxische Alkalisalz der Essigsäure in einer solchen Menge eingesetzt wird, dass das aus dem Acetat bei der Oxidation gebildete Bicarbonat 40 bis 120 mOsm pro Liter beträgt.

5. Veterinärzusammensetzung nach einem der Ansprüche 1 bis 4, dadurch gekennzeichnet, dass als Alkalisalz der Essigsäure Natriumacetat vorliegt.

6. Veterinärzusammensetzung nach einem der Ansprüche 1 bis 5, dadurch gekennzeichnet, dass Natrium- und Kaliumionen vorhanden sind und die Summe des vorhandenen Natriums und Kaliums 120 bis 160 mOsm/l beträgt, wobei Kalium zu 10 bis 30 mOsm/l und Natrium zu 110 bis 150 kOsm/l vorliegt.

7. Veterinärzusammensetzung nach einem der Ansprüche 1 bis 6, dadurch gekennzeichnet, dass die vorhandenen, aus dem Monosaccharid, der Aminosäure, der Zitronensäure und dem Acetat bestehenden Energiesubstrate beim Stoffwechsel 50 bis 100 Prozent des Energiebedarfs des Tieres liefern.

8. Veterinärzusammensetzung nach Anspruch 7, dadurch gekennzeichnet, dass beim Stoffwechsel der Energiesubstrate die verbleibenden Ionen angenähert eine iso-osmotische extrazelluläre Flüssigkeit bilden.

9. Veterinärzusammensetzung zur Verwendung bei der Behandlung von Diarrhoe bei Rindern, bestehend aus einer wässrigen Lösung eines aktiv resorbierten Monosaccharids, einer aktiv resorbierten, natürlich vorkommenden Aminosäure sowie Zitronensäure, dadurch gekennzeichnet, dass sie ferner ein nicht-toxisches Alkalisalz der Essigsäure enthält und bei der Oxidation eine Osmolatität von 50 bis 100 mOsm ergibt.

10. Veterinärzusammensetzung nach einem der Ansprüche 1 bis 9, dadurch gekennzeichnet, dass als Monosaccharid Glucose und als Aminosäure Glycin vorliegen.

**Revendications**

1. Une composition véterinaire facilement absorbée et métabolisée, utile pour le traitement de diarrhée chez les mammifères, et contenant une proportion majeur d'un monosaccharide absorbé activement et des proportions mineures d'un acide aminé d'origine naturelle absorbé activement et de l'acide citrique, caractérisée en ce que la composition contient en outre de 4 à 12 pour cent en poids d'un sel alcalin non toxique de l'acide acétique, la concentration en monosaccharide étant de 70 à 90 pour cent en poids, la concentration en acide aminé étant de 1 à 7,5 pour cent en poids et la concentration en acide citrique étant de 0,1 à 5 pour cent en poids, toutes étant basées sur le poids sec.

2. Une composition vétérinaire selon la revendication 1, caractérisée en ce qu'elle est dissoute dans un véhicule aqueux convenable pour une administration vétérinaire de telle manière que le monosaccharide soit présent à une concentration de 30 à 90 grammes par litre.

3. Une composition vétérinaire selon la revendication 2, caractérisée en ce que l'osmolatité du fluide est comprise entre 600 et 1.200 mOsm par litre.

4. Une composition vétérinaire selon la revendication 2 ou 3, caractérisée en ce que le sel alcalin non toxique de l'acide acétique est fourni en quantité telle que le bicarbonate formé à partir de l'acétate au fur et à mesure de son oxydation sera présent en quantité comprise entre 40 et 120 mOsm par litre.

5. Une composition vétérinaire selon l'une quelconque des revendications 1 à 4, caractérisée en ce que le sel alcalin de l'acide acétique est l'acétate de sodium.

6. Une composition vétérinaire selon l'une quelconque des revendications 1 à 5, caractérisée en ce que des ions sodium et potassium sont présents, la somme du sodium et du potassium présents est de 120 à 160 mOsm/l, le potassium étant présent à 10 à 30 mOsm/l et le sodium à 110 à 150 mOsm/l.

7. Une composition vétérinaire selon l'une quelconque des revendications 1 à 6, caractérisée en ce que les substrats énergétiques constitués du monosaccharide, de l'acide aminé, le l'acide citrique et de l'acétate présents fournissent de 50 à 100 pour cent des besoins énergétiques de l'animal au fur et à mesure qu'ils sont métabolisés.

8. Une composition vétérinaire selon la revendication 7, caractérisée en ce que au fur et à mesure que les substrats énergétiques sont métabolisés, les ions restants se rapprochent d'un fluide extracellulaire isoosmotique.

9. Une composition vétérinaire utile pour traiter la diarrhéa chez les animaux bovins et comprenant

une solution aqueuse d'un monosaccharide absorbé activement, d'un acide aminé d'origine naturelle absorbé activement et de l'acide citrique, caractérisée en ce qu'elle contient en outre un sel alcalin non toxique de l'acide acétique et qu'elle aura une osmolatité de 50 à 100 mOsm/l au fur et à mesure de son oxydation.

10. Une composition vétérinaire selon l'une quelconque des revendications 1 à 9, caractérisée en ce que le monosaccharide est le glucose et l'acide aminé est la glycine.

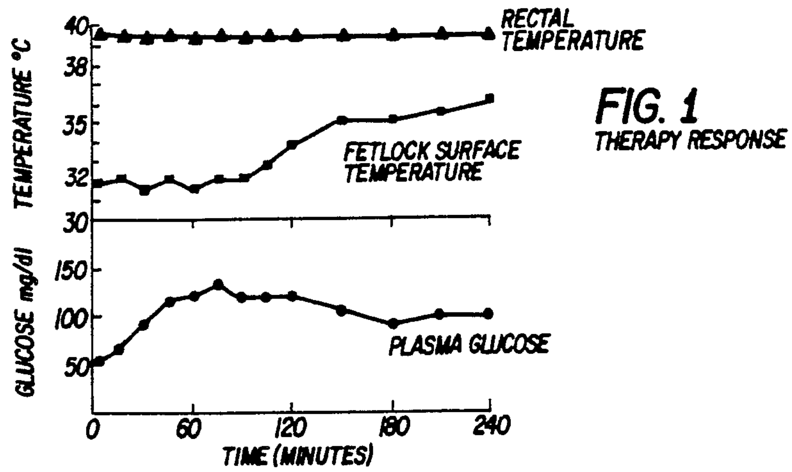


FIG. 1
THERAPY RESPONSE

FIG. 2
GLUCOSE BLOOD LEVELS BY TIME POST-DOSE

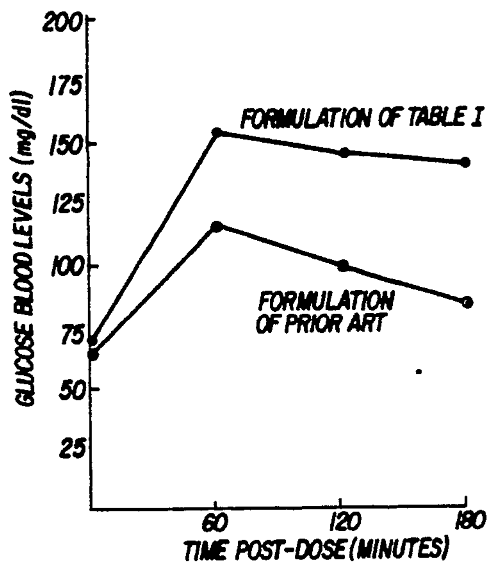


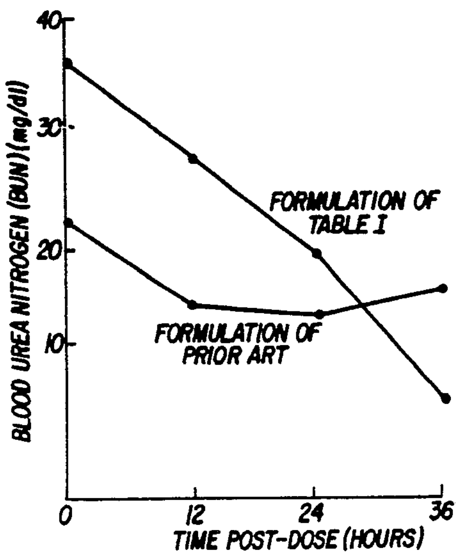


FIG. 3
CHANGE IN BUN DURING THE COURSE OF THERAPY

FIG. 4

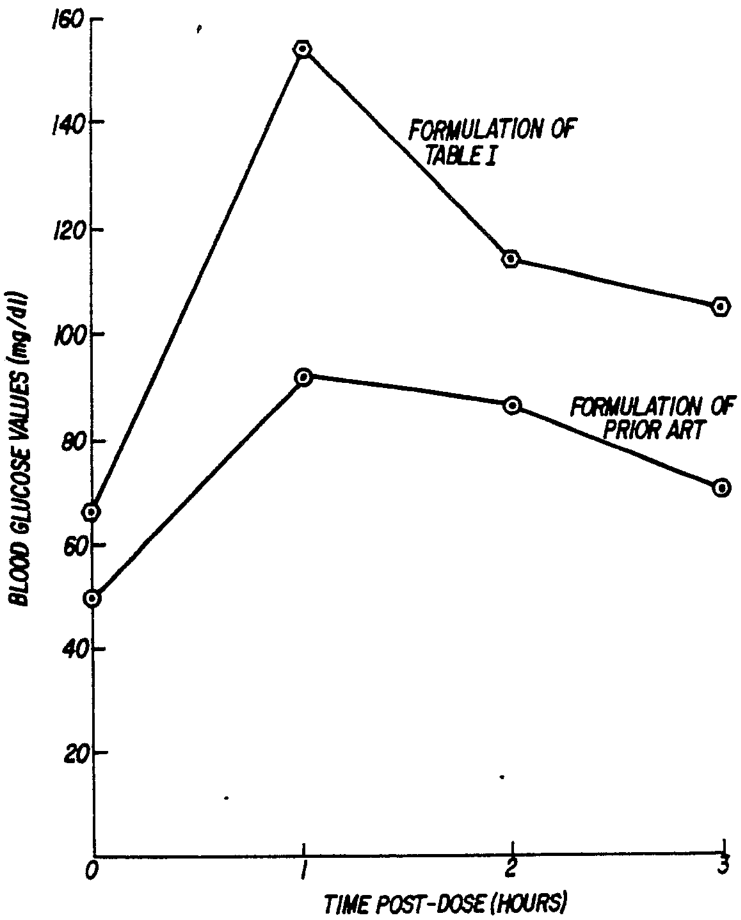
160
140
120
100
80
60
40
20
BLOOD GLUCOSE VALUES (mg/dl)
FORMULATION OF TABLE I
FORMULATION OF PRIOR ART
0
1
2
3
TIME POST-DOSE (HOURS)